# EUROPEAN PATENT SPECIFICATION

(11) **EP 4 140 257 B1**
(45) Date of publication and mention of the grant of the patent: **21.05.2025**
(21) Application number: 20720812.5
(22) Date of filing: 21.04.2020
(51) Int. Cl.: H05B 45/20, A61L 2/10, H05B 47/115, A61L 9/20, A61L 2/24

(54) **UV LIGHT CONTROL SYSTEM**
UV-LICHT-STEUERUNGSSYSTEM
SYSTÈME DE COMMANDE DE LUMIÈRE UV

(43) Date of publication of application: 01.03.2023
(73) Proprietor: BrainLit AB, 223 63 Lund (SE)
(72) Inventor: WINGREN, Tord, 217 74 Malmö (SE)
(74) Representative: Zacco Sweden AB
(86) International application number: PCT/EP2020/061071
(87) International publication number: WO 2021/213633

(56) References cited:
- US-A1- 2018 185 527
- US-A1- 2018 296 711
- US-A1- 2019 247 528
- US-A9- 2017 028 088

## Description

### Technical field

The present invention relates to a light control system. Especially for controlling UV light exposure to sub-portions of a space.

### Background of the invention

With the technical evolution of artificial light, much driven by the development of light emitting diodes, improved light sources are now in the hands of light designers. The light sources allow, for example, for mimicking of daylight in an indoor environment, but also offer new possibilities to use light within an environment. Ultraviolet light, for example, can be used to disinfect objects and surfaces. However, ultraviolet light is not only hazardous to microorganisms such as virus, bacteria and fungus, but it is also known that it can damage materials and surfaces present within a space which is irradiated.

US 2018/185527 A1 disclose control systems for disinfecting light systems and methods of regulating disinfecting energy generated by disinfecting systems are disclosed. The control system may include a first sensor and a second sensor positioned within a space illuminated by the disinfecting light system. The first sensor may measure an amount of disinfecting energy provided to the space by the disinfecting light system, and the second sensor may detect an environmental characteristic of the space. Additionally, the control system may include a controller operably coupled to the first and second sensor. The controller may regulate the disinfecting energy generated by the disinfecting light system by adjusting the amount of disinfecting energy provided to the space by the disinfecting light system in response to the amount of disinfecting energy provided to the space by the disinfecting light system measured by the first sensor, and/or the environmental characteristic detected by the second sensor.

US 2018/296711 A1 disclose room decontamination systems, controllers and methods for decontaminating a room. The room decontamination system may be a UV room decontamination system that uses UV radiation to perform a decontamination operation in the room. A controller may determine whether safe conditions for decontamination exist and initiate a decontamination operation on the basis of whether they exist. Determination of safe conditions for decontamination may be based on light actuation detection and/or sensor data, which may include presence detector data and door sensor data. Determination of safe conditions for decontamination may include a determination of whether sensors are functioning properly. The controller may also determine whether decontamination operations are required on the basis of the historical condition data, for example on the basis of whether the room has been occupied since the last decontamination operation.

In order to achieve improved lighting solutions capable of disinfecting surfaces and objects, there is a need for efficient control of light sources that deliver ultraviolet light to the surfaces and objects within the space.

### Summary of the invention

In view of the above, it is an object of the present invention to provide a light control system capable of illuminating a space with ultraviolet light in an efficient manner. It is a further object to mitigate, alleviate or eliminate one or more of the above-identified deficiencies in the art and disadvantages singly or in any combination and solve at least the above-mentioned problem.

The present invention provides a light control system according to claim 1 and a method according to claim 12. Preferred embodiments are defined in the dependent claims.

According to a first aspect a light control system is provided. The light control system comprising: a plurality of light sources each configured to emit light within an ultraviolet spectral range, each of the plurality of light sources being configured to illuminate a corresponding sub-portion of a space; a positioning system configured to determine positions of one or more individuals within the space; a density system configured to, based on by the positioning system over time determined positions of the one or more individuals, determine a density distribution of the one or more individuals in the space; and a control engine configured to control the plurality of light sources based on the density distribution such that a sub-portion of the space having a relatively high density is illuminated with light within the ultraviolet spectral range to a relatively higher degree than a sub-portion of the space having a relatively low density.

Within the context of this disclosure, the wording "light within an ultraviolet spectral range" may be construed as electromagnetic radiation having a wavelength within the ultraviolet spectral range. The wavelength range for ultraviolet spectral range is typically from 10 nm to 400 nm. It is realized that different parts of the ultraviolet spectral range may be more suitable to use in the present light control system than other parts. UV-C, for example, is well known as being suitable and efficient for disinfect surfaces/objects by killing and/or inactivating microorganisms.

Within the context of this disclosure, an individual may be a person or an animal.

Within the context of this disclosure, a "density distribution" may be construed as a distribution of how frequently positions of the space are visited/occupied by the one or more individuals. The density distribution may be represented by a heatmap, in which the degree of how frequently positions have been visited/occupied by the one or more individuals are indicated by, e.g., a color choice. Hence, the heat map illustrates how the density distribution varies over space.

Efficient illumination of the space with light within the ultraviolet spectral range is thereby allowed. It is appreciated that the present light control system allows an efficient use of the light sources, as the amount of ultraviolet light emitted by each light source is controlled such that the respective sub-portions of the space are illuminated depending on the degree that one or more individuals have been present in the sub-portion of the space, thereby allowing the light control system to reduce usage of light sources associated with sub-portions of the space that individuals occupy less frequently. By reducing the usage of the light sources, the light control system is allowed to reduce potential damages to surfaces and/or objects present in the space. Furthermore, by reducing the usage of the light sources, the associated wear of the light source is also reduced, thereby increasing a lifespan over which the light sources can be used.

The control engine may be further configured to control the plurality of light sources based on a present position of the one or more individuals within the space, such that a sub-portion of the space in which the one or more individuals are presently present is not exposed to the ultraviolet light emitted by one or more of the plurality of light sources.

A possible associated advantage is that the light control system may not illuminate sub-portions of the space that individuals are presently occupying the sub-portion since ultraviolet light may be hazardous to individuals. Hence, by configuring the control engine to control the light sources to expose sub-portions not presently occupied by an individual to ultraviolet light, a light control system that automatically adjusts the exposure of the space to ultraviolet light depending on a current position of the one or more individuals may be allowed.

A radiant flux of light within the ultraviolet spectral range emitted by each of the plurality of light sources may be adjustable, and wherein the control engine may be further configured to control the radiant flux of light within the ultraviolet spectral range emitted by the plurality of light sources such that each sub-portion of the space is given a respective UV light dose in a predetermined period of time.

By "radiant flux" herein is generally meant the energy of light emitted by a light source per unit time. Hence, "the radiant flux of light within the ultraviolet spectral range emitted by the plurality of light sources" is therefore meant to be construed as the energy of light within the ultraviolet spectral range emitted by the plurality of light sources.

A possible associated advantage is that each sub-portion of the space may be given a respective UV light dose in a predetermined period of time. This allows the light control system, e.g., to disinfect surfaces and/or objects in a sub-portion of the space in a predetermined period of time, e.g. an hour or overnight.

The light control system may further comprise: a distance sensor configured to determine a distance between a light source of the plurality of light sources and an object present within the respective sub-portion of the space; and wherein the control engine may be further configured to control the plurality of light sources based on the determined distance between each light source and the object present within each respective sub-portion of the space, such that an object at a relatively longer distance from the light source is illuminated by the light source to a relatively higher degree than an object at a relatively shorter distance.

A possible associated advantage is that an intensity of ultraviolet light that the object in the sub-portion of the space is exposed to may be adjusted. Further, by accounting for the distance between the light source and the object, the control engine may control the light sources such that the object is given the predetermined UV light dose over time.

The light control system may further comprise: an object sensor configured to determine a property of an object present within the space, the property being associated with a sensitivity to ultraviolet light and/or an expected contamination level; and wherein the control engine may be further configured to control the plurality of light sources based on the determined property of the object, such that an object having a relatively low sensitivity to ultraviolet light and/or a relatively high expected contamination level is illuminated with light within the ultraviolet spectral range to a relatively higher degree than an object having a relatively high sensitivity to ultraviolet light and/or a relatively low expected contamination level.

A possible associated advantage is that objects having a relatively high sensitivity to light within ultraviolet spectral range may be given a relatively lower UV light dose over time, thereby reducing a risk of damaging the object through exposure to ultraviolet light.

A further possible associated advantage is that objects having a high expected contamination level may be given a relatively high UV light dose, thereby disinfecting objects having a relatively high expected contamination level to a relatively higher degree than objects having a relatively lower expected contamination level.

Hence, by taking the property of the object into account, a UV light dose to be given to the object over time may be adjusted.

The light control system may further comprise: a pathogen sensor configured to dynamically determine a presence of a pathogen within the space; and wherein the control engine may be further configured to control each of the plurality of light sources based on the determined presence of the pathogen within each respective sub-portion of the space, such that a sub-portion of the space having a determined presence of the pathogen is exposed to light within the ultraviolet spectral range to a relatively higher degree than a sub-portion of the space not having a determined presence of the pathogen.

A possible associated advantage is that sub-portions having a determined presence of the pathogen may be exposed to a relatively higher degree than sub-portions of the space not having a determined presence of the pathogen. Hence, a more power efficient use of the light sources may be allowed.

Each light source of the plurality of light sources may be configured to communicate illumination data to the control engine, and the illumination data comprising data of a radiant flux of light within the ultraviolet spectral range emitted by the light source, and wherein the control engine may be further configured to determine, by integrating or accumulating the over time received illumination data, a UV light dose given to each corresponding sub-portion of the space.

A possible associated advantage is that the UV light dose given to each corresponding sub-portion of the space may be determined without using a light sensor sensitive to light within the ultraviolet spectral range. Hence, a less complex and more cost-efficient light control system may thereby be allowed.

The light control system may further comprise: an image sensor arranged to acquire one or more images of the space; and wherein the control engine may be further configured to: using an image acquired by the image sensor, determine, within the space, an illumination distribution of light within the ultraviolet spectral range; and determine, based on the over time determined illumination distribution, a UV light dose distribution given to the space.

A possible associated advantage is that spatial and/or granular information of the UV light dose given to the space may be available to the control engine, thereby allowing the control engine to determine how the UV light dose given to certain parts of a sub-portion relates to a predetermined UV light dose to be given to each part of the sub-portion over time.

The density system may be configured to discard determined positions being older than a predetermined age.

A possible associated advantage is that the density system may determine a density distribution based on positions being younger than the predetermined age, thereby allowing the light control system to control the plurality of light sources such that sub-portions of the space which the one or more individuals have not recently visited are illuminated with light within the ultraviolet spectral range to a relatively lower degree than sub-portions of the space which the one or more individuals have recently visited. Hence, only sub-portions of the space that the one or more individuals have recently visited may be disinfected. The plurality of light sources may thereby be used more efficiently, whereby an associated wear and tear of each light source may be reduced. Hence, the plurality of light sources may continue to function, and thereby be used, for a longer period of time.

The predetermined age may be set based on a UV light dose given to the space.

A possible associated advantage is that the density system may determine a density distribution further based on the UV light dose given to the space, thereby allowing the light control system to control the plurality of light sources based on the UV light dose given to the space. An associated wear and tear of each light source may thereby be reduced, whereby the plurality of light sources may continue to function, and thereby be used, for a longer period of time.

The predetermined age for each sub-portion of the space may be set based on a UV light dose given to each corresponding sub-portion of the space.

A possible associated advantage is that the density system may determine a density distribution further based on the UV light dose given to the sub-portions of the space, thereby allowing the light control system to control each light source based on the UV light dose given to the respective sub-portion of the space. An associated wear and tear of each light source may thereby be reduced, whereby the plurality of light sources may continue to function, and thereby be used, for a longer period of time.

According to a second aspect, a method for illuminating a space with light within an ultraviolet spectral range using a plurality of light sources each configured to illuminate a corresponding sub-portion of the space is provided. The method comprising: determining positions of one or more individuals within the space; determining a density distribution based on the over time determined positions of the one or more individuals in the space; and illuminating the space by the plurality of light sources based on the density distribution such that a sub-portion of the space having a relatively high density is illuminated with light within the ultraviolet spectral range to a relatively higher degree than a sub-portion of the space having a relatively low density.

The above-mentioned features of the light control system, when applicable, apply to this second aspect as well. In order to avoid undue repetition, reference is made to the above.

A further scope of applicability of the present disclosure will become apparent from the detailed description given below. However, it should be understood that the detailed description and specific examples, while indicating preferred variants of the present inventive concept, are given by way of illustration only, since various changes and modifications within the scope of the inventive concept will become apparent to those skilled in the art from this detailed description.

Hence, it is to be understood that this inventive concept is not limited to the particular steps of the methods described or component parts of the systems described as such method and system may vary. It is also to be understood that the terminology used herein is for purpose of describing particular embodiments only and is not intended to be limiting. It must be noted that, as used in the specification and the appended claim, the articles "a", "an", "the", and "said" are intended to mean that there are one or more of the elements unless the context clearly dictates otherwise. Thus, for example, reference to "a unit" or "the unit" may include several devices, and the like. Furthermore, the words "comprising", "including", "containing" and similar wordings do not exclude other elements or steps.

### Brief description of the drawings

The above and other aspects of the present inventive concept will now be described in more detail, with reference to appended drawings showing variants of the invention. The figures should not be considered limiting the invention to the specific variant; instead they are used for explaining and understanding the inventive concept.

As illustrated in the figures, the sizes of layers and regions are exaggerated for illustrative purposes and, thus, are provided to illustrate the general structures of variants of the present inventive concept. Like reference numerals refer to like elements throughout.
Figure 1A illustrates a light control system configured to control an illumination of a space.
Figure 1B illustrates a side-view of a sub-portion of the space.
Figure 2A illustrates an example scenario in which one or more individuals moves in a space.
Figure 2B illustrates a representation of a density distribution of the scenario of Fig. 2A.
Figure 3A illustrates a side-view of two sub-portions of a space where an individual is presently present in one of the sub-portions.
Figure 3B illustrates the scenario in Fig. 3A when the individual has left a sub-portion of the space which is then exposed to ultraviolet light.
Figure 4 is a block scheme of a method for illuminating a space with light within an ultraviolet spectral range.

### Detailed description

The present inventive concept will now be described more fully hereinafter with reference to the accompanying drawings, in which currently preferred variants of the inventive concept are shown. This inventive concept may, however, be implemented in many different forms and should not be construed as limited to the variants set forth herein; rather, these variants are provided for thoroughness and completeness, and fully convey the scope of the present inventive concept to the skilled person.

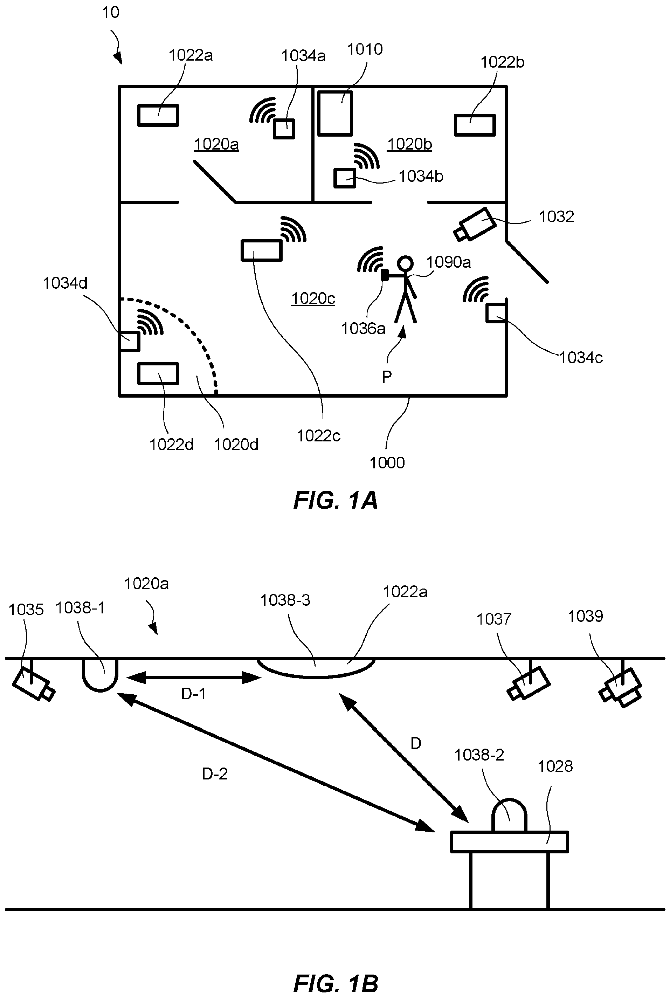

A light control system 10 will now be described with reference to Fig. 1 - Fig. 4.

Figure 1A illustrates the light control system 10. The light control system 10 comprises a plurality of light sources 1022, a positioning system, a density system, and a control engine 1010. The plurality of light sources 1022 shown in the example of Fig. 1A comprises a first light source 1022a, a second light source 1022b, a third light source 1022c, and a fourth light source 1022d. Each light source of the plurality of light sources 1022 comprises at least one luminaire. Each light source of the plurality of light sources 1022 may comprise a plurality of luminaires. The plurality of light sources 1022, the positioning system, and the density system may be configured to communicate with the control engine 1010. The communication may be implemented using wired or wireless technologies, or a combination of wired and wireless technologies. One or more of the plurality of light sources 1022, the positioning system, the density system, and the control engine 1010 may be further configured to communicate with a server (not shown). The server may be arranged at a different physical location. The communication may be via an external network, e.g. the Internet. The server may provide software updates to the light control system 10. The server may perform backups of the light control system 10, e.g., by copying data stored on the control engine 1010 to the server.

Each light source of the plurality of light sources 1022 is configured to emit light within an ultraviolet spectral range. The ultraviolet spectral range may be within a UV-C spectral range. UV-C light may provide efficient prevention of bacteria. In other words, UV-C may be germicidal. UV-C may deactivate bacteria, viruses and other pathogens and thereby destroy their ability to multiply and cause disease. Specifically, UV-C light may cause damage to the nucleic acid of microorganisms by forming covalent bonds between certain adjacent bases in the DNA. Each light source of the plurality of light sources 1022 is further configured to illuminate a corresponding sub-portion 1020 of a space 1000. In the example shown in Fig. 1A, the first light source 1022a is configured to illuminate a first sub-portion 1022a of the space 1000, the second light source is configured to illuminate a second sub-portion 1022b of the space 1000, the third light source 1022c is configured to illuminate a third sub-portion 1022c of the space 1000, and the fourth light source 1022d is configured to illuminate a fourth sub-portion 1020d of the space 1000. The light sources 1022a, 1022b, 1022c, 1022d may be configured to stop illuminating each corresponding sub-portion 1020a, 1020b, 1020c, 1020d of the space 1000 with light within the ultraviolet spectral range in case a communication between the light source 1022a, 1022b, 1022c, 1022d and the control engine 1010 is interrupted. A sub-portion 1020 of the space 1000 may be a separate room, which is exemplified in Fig. 1A by the first sub-portion 1020a and the second sub-portion 1020b. A sub-portion 1020 of the space 1000 may be a part of a room, which is exemplified by the third sub-portion 1020c and fourth sub-portion 1020d, which are parts of a same room within the space 1000. One or more sub-portions 1020 may be overlapping, i.e. more than one light source 1022 may be arranged to illuminate at least an overlapping part of two sub-portions 1020. The plurality of light sources 1022 may be further configured to emit light within a visible spectral range. The visible spectral range may be from 400 nm to 750 nm. The plurality of light sources 1022 may thereby illuminate corresponding sub-portions 1020 of the space 1000 with visible light.

The positioning system is configured to determine positions P of one or more individuals 1090 within the space 1000. The positioning system will now be described with reference to a single individual 1090a of the one or more individuals 1090, however it is to be understood that the positioning system may be configured to determine positions of each of the one or more individuals 1090 in a similar manner. The positioning system may, as exemplified in Fig. 1A, comprise one or more optical detectors 1032 configured to communicate with the control engine 1010, each optical detector 1032 being associated with a position within the space 1000. The one or more optical detectors 1032 may be configured to detect the individual 1090a, thereby determining a position P the individual 1090a. The position P of the individual 1090a may thereby be determined without a need for the individual 1090a to carry a personal identification device. The positioning system may be further configured to identify the individual 1090a. The positioning system may comprise processing capabilities configured to identify the individual 1090a, and to communicate the identity of the individual 1090a to the control engine 1010. The individual 1090a may be identified by facial recognition. The one or more optical detectors 1032 may comprise one or more cameras and/or one or more image sensors. The one or more optical detectors 1032 may comprise one or more Modcams. A Modcam is an optical detector that uses computer vision to determine movements and profiles of individuals 1090 and may thereby be used to identify and/or track the individual 1090a within the space 1000. The one or more optical detectors 1032 may be configured to communicate with the control engine 1010 via a wired and/or wireless connection. Each one or more optical detectors 1032 may be configured to determine a position of the individual 1090a relative to respective optical detector 1032, and the position P of the individual 1090a may be determined based on the position associated with each optical detector 1032 and the determined position the individual 1090a relative to respective optical detector 1032.

The positioning system may comprise, as exemplified in Fig. 1A, one or more electronic devices 1034, each electronic device 1034 being associated with a position within the space 1000, and a portable device 1036 associated with the individual 1090a, the portable device 1036 being configured for wireless communication with the one or more electronic devices 1034. The position P of the individual 1090a may be determined based on the wireless communication between the one or more electronic devices 1034 and the portable device 1036 of the individual 1090a. The position P of the individual 1090a may thereby be determined in a less complex and more reliable manner. The one or more electronic devices 1034 may comprise a Bluetooth beacon and/or a WIFI access point. The one or more electronic devices 1034 may be comprised in the one or more light sources 1022, as exemplified in Fig. 1A with light sources 1022c located in the third sub-portion 1020c. The portable device 1036 may be a wireless token or a wireless tag. The portable device 1036 may be a smartphone, a smartwatch, a tag, a smartring, or a wireless key. The portable device 1036 and the one or more electronic devices 1034 may communicate via WIFI, Bluetooth, RFID and/or NFC. The wireless communication between the portable device 1036 and the one or more electronic devices 1034 may be based on ultra-wideband (UWB) technology, which may allow for a reliable and more precise determination of the position P of the individual 1090a. The position P of the individual 1090a may be based on a number of electronic devices 1034 in communication with the portable device 1036. The position P of the individual 1090 may be based on a signal strength, angle-of-arrival information, and/or time-of-arrival, associated with the wireless communication between the one or more electronic devices 1034 and the portable device 1036. Thus, the position P of the individual 1090a may be determined based on the position associated with each of the one or more electronic devices 1034 and relative positions between the portable device 1036 and the one or more electronic devices 1034 in communication with the portable device 1036.

The one or more electronic devices 1034 may be further configured for wireless communication with the control engine 1010, and the control engine 1010 may be further configured to determine the position P of the individual 1090a based on the wireless communication between the one or more electronic devices 1034 and the portable device 1036. The portable device 1036 carried by the individual 1090a may thereby not need processing capabilities, and thus having a reduced power consumption. Thus, any necessary calculations may be performed by the control engine 1010.

The portable device 1036 may be further configured to communicate with the control engine 1010, and to determine the position P of the individual 1090a based on the wireless communication between the one or more electronic devices 1034 and the portable device 1036. The portable device 1036 may be configured to communicate with control engine 1010 via a wireless technology, for example WIFI, Bluetooth, and/or NFC. The portable device 1036 may be configured to communicate with the control engine 1010 via an internet connection. The portable device 1036 may comprise a processor configured to determine the position P of the individual 1090a based on the wireless communication between the one or more electronic devices 1034 and the portable device 1036. Processing related to the determination of the position P of each of the one or more individuals 1090 may thereby be distributed to a plurality of portable devices 1036, thus reducing processing load of the control engine 1010.

The control engine 1010 may be configured to determine in which sub-portion 1020 of the space 1000 the individual 1090a is presently located based on the determined position P of the individual 1090a. In the example shown in Fig. 1, the control engine 1010 determines that the individual 1090a is presently located in the third sub-portion 1020c based on the determined position P of the individual 1090a.

The positioning system is configured to, over time, determine a plurality of positions P of the individual 1090a within the space 1000. The positioning system may be configured to continuously determine the position P of the individual 1090a. Alternatively, or additionally, the positioning system may be configured to intermittently determine the position P of the individual 1090a. The positioning system may be configured to continuously and/or intermittently communicate the determined positions P of the individual 1090a within the space 1000 to the control engine 1010.

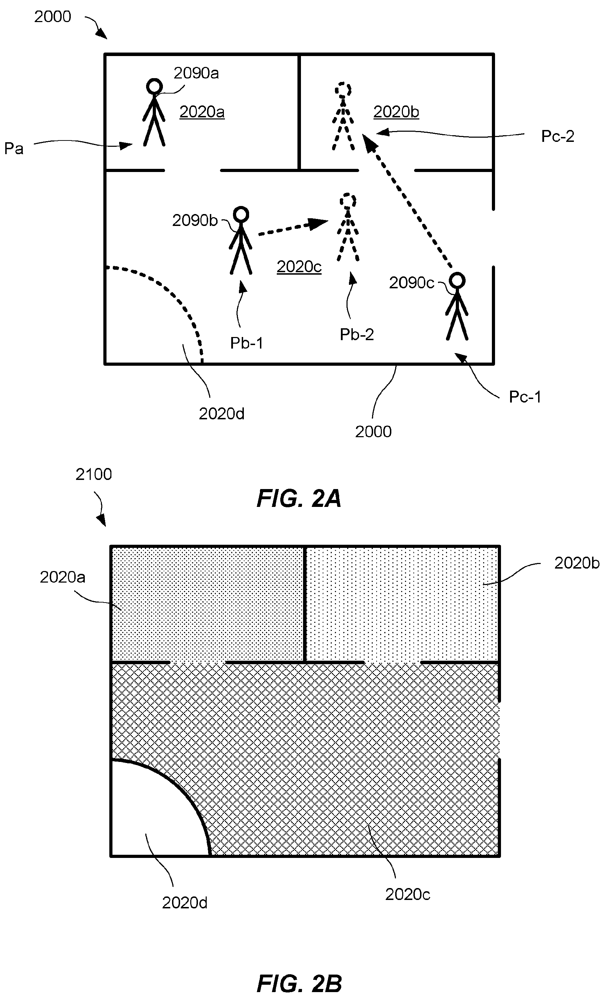

The density system is configured to, based on by the positioning system over time determined positions P of the one or more individuals 1090, determine a density distribution of the one or more individuals 1090 in the space 1000. An example of a density distribution will be described in connection with Fig. 2A. The density system may comprise computational capabilities. The density system may comprise a computer and/or a server. The density system may be implemented by the control engine 1010, i.e. the functions of the density system may be performed by the control engine 1010. The density distribution may correspond to the space 1000. The density distribution may be two-dimensional, i.e. it may correspond to the surface of the space 1000. The density distribution may be represented by a heatmap of the space 1000. The density distribution may be determined by integrating the over time determined positions P of the one or more individuals 1090. Hence, the density distribution indicates an extent to which the one or more individuals 1090 have been at different positions within the space 1000.

The control engine 1010 is configured to control the plurality of light sources 1022 based on the density distribution such that a sub-portion 1020 of the space 1000 having a relatively high density is illuminated with light within the ultraviolet spectral range to a relatively higher degree than a sub-portion 1020 of the space 1000 having a relatively low density. The control engine 1010 may comprise processing capabilities, e.g. a central processing unit. The control engine 1010 may further comprise a non-transitory computer-readable storage medium.

The control engine 1010 may be configured to determine information correlating positions in the space 1000 to sub-portions 1020 of the space 1000. Alternatively, or additionally, the non-transitory computer-readable storage medium of the control engine 1010 may comprise information correlating positions in the space 1000 to sub-portions 1020 of the space 1000. A position of the space 1000 may be correlated to a sub-portion 1020 of the space 1000 by using a position and/or illumination properties (e.g. divergence and direction of the illumination cone of light emitted from the light source 1022, the height above the floor at which the light source 1022 is mounted etc.) of each of the plurality of light sources 1022. Thus, the control engine 1010 may have access to information correlating each position in the space 1000 to a sub-portion 1020 of the space 1000, and since each of the plurality light sources 1022 illuminates a corresponding sub-portion 1020 of the space 1000, the control engine 1010 may be configured to control the plurality of light sources 1022 by comparing the density distribution with the information correlating each position in the space 1000 to a sub-portion 1020 of the space 1000.

Efficient illumination of the space 1000 with light within the ultraviolet spectral range is thereby allowed. It is appreciated that the light control system 10 allows an efficient use of the plurality of light sources 1022, as the amount of ultraviolet light emitted by each light source 1022 is controlled such that the respective sub-portions 1020 of the space 1000 are illuminated depending on the degree that one or more individuals 1090 have been present in the sub-portion 1020 of the space 1000, thereby allowing the light control system 10 to reduce usage of light sources 1022 associated with sub-portions 1020 of the space 1000 that individuals 1090 have occupied less frequently. By reducing the usage of the light sources 1022, the light control system 10 is allowed to reduce potential damages to surfaces and/or objects present in the space 1000. Furthermore, by reducing the usage of the light sources 1022, the associated wear of the light sources 1022 is also reduced, thereby increasing a lifespan over which the light sources 1022 can be used.

The control engine 1010 may be further configured to control the plurality of light sources 1022 based on a present position P of the one or more individuals 1090 within the space 1000, such that a sub-portion 1020 of the space 1000 in which the one or more individuals 1090 are presently present is not exposed to the ultraviolet light emitted by one or more of the plurality of light sources 1022. Hence, in the example shown in Fig. 1A, the light control system 10 may be configured to not illuminate sub-portion 1020c of the space 1000, as the individual 1090a is presently present in that sub-portion 1020c and it may be hazardous for the individual 1090a to be exposed to ultraviolet light.

A radiant flux of light within the ultraviolet spectral range emitted by each of the plurality of light sources 1022 may be adjustable. The control engine 1010 may be further configured to control the radiant flux of light within the ultraviolet spectral range emitted by the plurality of light sources 1022 such that each sub-portion 1020 of the space 1000 is given a respective UV light dose in a predetermined period of time. This may allow the light control system 10, e.g., to disinfect surfaces and/or objects in a sub-portion 1020 of the space 1000 in a predetermined period of time, e.g. an hour or overnight. The radiant flux of light within the ultraviolet spectral range emitted by each light source 1022 may be adjusted based on an age and/or usage of the respective light source 1022. It is known within the art that the radiant flux of light within the ultraviolet spectral range emitted by a light source is reduced as the light source ages (e.g., by being used). Hence, by taking the age and/or usage of each light source 1022 into account, the control engine 1010 may ensure that the UV light dose given to a sub-portion 1020 of the space 1000 over time is above a predetermined threshold. For example, as the radiant flux of light within the ultraviolet spectral range emitted by a light source 1022 may degrade over time as the light source 1022 is aged/used, the control engine 1010 may increase the radiant flux in order to compensate for the degradation of the light source 1022. The control engine 1010 may be further configured to control the radiant flux of light within the ultraviolet spectral range emitted by each of the plurality of light sources 1022 based on the density distribution such that a sub-portion 1020 of the space 1000 having a relatively high density may be illuminated with light within the ultraviolet spectral range with a relatively higher radiant flux than a sub-portion 1020 of the space 1000 having relatively low density. The predetermined UV light dose may be set such that each respective sub-portion 1020 of the space 1000 is disinfected, e.g., by reducing pathogens present in the sub-portion 1020 of the space 1000. The radiant flux for light within the ultraviolet spectral range emitted by each of the plurality of light sources 1022 may be further based on properties (e.g. reflectivity, material, etc.) of objects (e.g., a floor, walls, furniture, etc.) present in each respective sub-portion 1020 of the space 1000.

The light control system 10 will now be described with further reference to Fig. 1B. Figure 1B illustrates a side-view of the first sub-portion 1020a of the space 1000. The first sub-portion 1020a illustrated in Fig. 1B correspond to the first sub-portion 1020a illustrated in Fig. 1A. In the example of Fig. 1B, the first light source 1022a is shown as being arranged in a ceiling of the first sub-portion 1020a and configured to illuminate in a direction toward the floor of the first sub-portion 1020a. However, it is to be understood that this is an example only, and that the light source 1022 may, e.g., be arranged at an inner wall or on a floor of the sub-portion 1020. Further, the light source 1022 may be stationary, as shown in the example of Fig. 1B, or movable. The light source 1022 may hence be a movable lamp, e.g. a movable floor lamp. A position of the movable lamp, and hence a position of the corresponding sub-portion 1020 which the movable lamp is configured to illuminate, may be determined by, e.g., the positioning system. The movable lamp may, e.g., comprise an electronic device configured to communicate with the one or more electronic devices 1034 of the position system. Alternatively, or additionally, the position of the movable lamp may be set and communicated to the control engine 1010 manually, e.g. by the use of the a front-end of the control engine 1010 or a computing device in communication with the control engine 1010 (e.g. a computer, a smartphone etc.).

Further, a direction in which the light source 1022 emits light may be adjustable. For example, the light source 1022 may be configured to illuminate a portion of a ceiling and/or a portion of a wall of the sub-portion 1020. The direction may, e.g., be adjustable manually or by the control engine 1010. The light source 1022 may be further configured to communicate the direction in which it emits light to the control engine 1010. The control engine 1010 may thereby be allowed to better determine a position of the corresponding sub-portion 1020 which the light source 1022 is configured to illuminate.

As shown in the example of Fig. 1B, the light control system 10 may further comprise a distance sensor 1038. The distance sensor 1038 may be configured to determine a distance between a light source 1022 of the plurality of light sources 1022 and an object 1028 present within the respective sub-portion 1020 of the space 1000. The light control system 10 may further comprise a plurality of distance sensors, shown in the example of Fig. 1B by the first distance sensor 1038-1, the second distance sensor 1038-2, and the third distance sensor 1038-3. The first distance sensor 1038-1 and the second distance sensor 1038-2 in this example are standalone devices, while the third distance sensor 1038-3 is integrated in a light source 1022 (e.g., a luminaire). It is to be understood that the one or more distance sensors 1038 may be integrated in other devices. The one or more distance sensors 1038 may be arranged in a plurality of different positions. This is exemplified in Fig. 1B by the first distance sensor 1038-1 being arranged in the ceiling, and the second distance sensor 1038-2 being arranged on a table.

The distance sensor 1038 may comprise an electromagnetic detector. The distance sensor 1038 may comprise a passive sensor, e.g. an image sensor. The distance sensor 1038 may be an active sensor comprising an emitter and a detector, e.g. a radar or a LIDAR. Hence, the distance sensor 1038 may be an active sensor configured to emit electromagnetic radiation and to detect reflections of the emitted electromagnetic radiation. The distance sensor 1038 may, e.g., determine the distance by determining a time difference between the emission of electromagnetic radiation and the detection of a reflection of the emitted electromagnetic radiation. Such technologies may be referred to as time-of-flight (TOF). Several different technologies that may be suitable for the distance sensor 1038 exists. The emitted electromagnetic radiation may be modulated in time, and by detecting modulations in the reflections of the emitted modulated electromagnetic radiation, the distance may be determined.

The distance sensor 1038 may directly determine the distance D between the light source 1022 and the object 1028, e.g., by being integrated in the light source 1022. This is exemplified in Fig. 1B by the third distance sensor 1038-3 being integrated in the light source 1022. The distance sensor 1038 may directly determine D the distance between the light source 1022 and the object 1028, e.g., by being integrated with or placed nearby the object 1028. This is exemplified in Fig. 1B by the second distance sensor 1038-2 being placed on the object 1028 (in this example being a table). The distance sensor 1038 may indirectly determine the distance D between the light source 1022 and the object 1028, e.g., by determining a first distance D-1 and direction between the distance sensor 1038-1 and the light source 1022, and a second distance D-2 and direction between the distance sensor 1038-1 and the object 1028. This is exemplified in Fig. 1B by the first distance sensor 1038-1.

The control engine 1010 may be further configured to control the plurality of light sources 1022 based on the determined distance D between each light source 1022 and the object 1028 present within each respective sub-portion 1020 of the space 1000, such that an object at a relatively longer distance from the light source 1022 is illuminated by the light source 1022 to a relatively higher degree than an object at a relatively shorter distance. An intensity of ultraviolet light that the object 1028 is exposed to may depend on the distance between light source 1022 and the object 1028. For example, since light emitted by the light source 1022 typically diverges, an intensity of ultraviolet light that the object 1028 in the sub-portion 1020 of the space 1000 is exposed to may be adjusted by adjusting the radiant flux and knowing the distance between the light source 1022 and the object 1028. The control engine 1010 may be configured to adjust the radiant flux for light within the ultraviolet spectral range emitted by each of the plurality of light sources 1022 further based on distances between each of the plurality of light sources 1022 and the objects within each sub-portion 1020 of the space 1000. Thus, by taking the distance between a light source 1022 and an object 1028 into account, the control engine 1010 may control the intensity of light within the ultraviolet spectral range at the object 1028. Hence, by accounting for the distance between the light source 1022 and the object 1028, the control engine 1010 may control the light sources 1022 such that the object 1028 is given the predetermined UV light dose over time.

The light control system 10 may further comprise an object sensor 1037. The distance sensor 1038 and the object sensor 1037 may be the same sensor. The object sensor 1037 may comprise an image sensor. The object sensor 1037 may, e.g., comprise a camera as exemplified in Fig. 1B. The object sensor 1037 may comprise computational capabilities. The object sensor 1037 may be configured to determine a property of an object 1028 present within the space 1000, the property being associated with a sensitivity to ultraviolet light and/or an expected contamination level. The property may be a material and/or a size of the object 1028 within the sub-portion 1020 of the space 1000.

The control engine 1010 may be further configured to control the plurality of light sources 1022 based on the determined property of the object 1028, such that an object having a relatively low sensitivity to ultraviolet light and/or a relatively high expected contamination level is illuminated with light within the ultraviolet spectral range to a relatively higher degree than an object having a relatively high sensitivity to ultraviolet light and/or a relatively low expected contamination level. Thus, objects having a relatively high sensitivity to light within ultraviolet spectral range may thereby be given a relatively low UV light dose over time, whereby a risk of damaging the object 1028 through exposure to ultraviolet light may be reduced. Alternatively, or additionally, objects having a high expected contamination level (e.g., plates, cutlery, medical tools, tables etc.) may thereby be given a relatively high UV light dose, whereby objects having a relatively high expected contamination level may be disinfected to a relatively higher degree than objects having a low expected contamination level. Thus, by taking the property of the object 1028 into account, a UV light dose to be given to the object 1028 over time may be adjusted accordingly.

The light control system 10 may further comprise a pathogen sensor 1039. The pathogen sensor 1039 may be the same sensor as the distance sensor 1038 and/or the object sensor 1037. The pathogen sensor 1039 may comprise computational capabilities. The pathogen sensor 1039 may be configured to dynamically determine a presence of a pathogen within the space 1000. The pathogen may be present on surfaces within the space 1000. The pathogen may be bacteria, virus, fungus etc. The pathogen sensor 1039 may be a passive sensor. The pathogen sensor 1039 may comprise one or more of an infrared detector, a thermal sensor, a spectrometer, a laser, or a thermal camera. In the example shown in Fig. 1B, the pathogen sensor 1039 is a thermal camera. The pathogen sensor 1039 may be an active sensor. The pathogen sensor 1039 may comprise an emitter configured to emit electromagnetic radiation and a detector configured to detect a pathogen that has been exposed to the emitted electromagnetic radiation. The detector of the pathogen sensor 1039 may detect electromagnetic radiation reflected by a surface, and, based on the intensity and/or spectrum of the reflected electromagnetic radiation, the detector may detect the presence of the pathogen. Alternatively, or additionally, the detector of the pathogen sensor 1039 may detect a fluorescence of the pathogen induced by the emitted electromagnetic radiation.

The control engine 1010 may be further configured to control each of the plurality of light sources 1022 based on the determined presence of the pathogen within each respective sub-portion 1020 of the space 1000, such that a sub-portion 1020 of the space 1000 having a determined presence of the pathogen is exposed to light within the ultraviolet spectral range to a relatively higher degree than a sub-portion 1020 of the space 1000 not having a determined presence of the pathogen.

Each sub-portion 1020 of the space 1000 may, over time, be given a respective predetermined UV light dose which is set based on the determined presence of the pathogen. The predetermined UV light dose may be set such that the pathogen is reduced or removed. In other words, the predetermined UV light dose may be set such that the sub-portion 1020 is disinfected after being exposed to ultraviolet light for the predetermined period of time. Thus, sub-portions 1020 having a determined presence of the pathogen may be exposed to ultraviolet light to a relatively higher degree than sub-portions 1020 of the space 1000 not having a determined presence of the pathogen. Hence, a more power efficient use of the light sources 1022 may be allowed. Surfaces and/or objects within sub-portions 1020 of the space 1000 not having a determined presence of the pathogen may thereby be exposed to ultraviolet light to a relatively low degree, whereby they are less likely to be damaged by the exposure to ultraviolet light.

The pathogen sensor 1039 may alternatively or additionally be configured to dynamically determine an amount of the pathogen within the space 1000. The control engine 1010 may alternatively or additionally be configured to control each of the plurality of light sources 1022 based on the determined amount of the pathogen with each sub-portion 1020 of the space 1000. In other words, each sub-portion 1020 of the space 1000 may receive a respective predetermined UV light dose which is set based on the determined amount of the pathogen. In other words, the UV light dose may be set such that the sub-portion 1020 is disinfected after being exposed to ultraviolet light for the predetermined period of time. The pathogen sensor 1039 may be configured to determine a relative amount of pathogen present in the sub-portion 1020 of the space 1000. The relative amount of pathogen may be determined by comparing over time determined signals being proportional to the amount of pathogen present in the sub-portion 1020 of the space 1000. For example, the pathogen sensor 1039 may determine that a pathogen is present in the sub-portion 1020 without determining an absolute amount of pathogen present in the sub-portion 1020. The pathogen sensor 1039 may subsequently determine a gradual decrease of the amount of pathogen as the light source 1022 exposes the sub-portion 1020 to ultraviolet light, thereby reducing the amount of pathogen present in the sub-portion 1020.

It is to be understood that positions of one or more of the distance sensor 1038, the object sensor 1037, the pathogen sensor 1039, and the image sensor 1035 may vary. In Fig. 1B, their positions are shown to be primarily in the ceiling of the sub-portion 1020a, however, it is to be understood that they may be arranged on a wall and/or a floor of the sub-portion 1020a. Two or more of the distance sensor 1038, the object sensor 1037, the pathogen sensor 1039, and the image sensor 1035 may further be comprised in a combined unit (not shown).

In a further example, one or more of the distance sensor 1038, the object sensor 1037, the pathogen sensor 1039, and the image sensor 1035 may be comprised in a movable light source, e.g., the movable lamp discussed previously. Further, it is envisioned that such a movable lamp may further comprise a control engine 1010, a positioning system, and a density system. Hence, such a movable lamp may be configured to independently perform one or more of the functions of the light control system 10.

Each light source of the plurality of light sources 1022 may be configured to communicate illumination data to the control engine 1010. The illumination data may comprise data of a radiant flux of light within the ultraviolet spectral range emitted by the light source. The control engine 1010 may be further configured to determine, by integrating or accumulating the over time received illumination data, a UV light dose given to each corresponding sub-portion 1020 of the space 1000. In other words, the UV light dose may be the radiant flux of light within the ultraviolet spectral range given to a sub-portion 1020 of the space 1000 integrated or accumulated for the predetermined period of time.

The light control system 10 may further adjust each light source 1022 based on the received illumination data. For example, to compensate for an aging light source.

The light control system 10 may further comprise an image sensor 1035 arranged to acquire one or more images of the space 1000. The image sensor 1035 may comprise an optical filter configured to transmit light having a wavelength within the ultraviolet spectral range. The image sensor 1035 may be comprised in a camera, as exemplified in Fig. 1B.

The control engine 1010 may be further configured to using an image acquired by the image sensor 1035, determine, within the space 1000, an illumination distribution of light within the ultraviolet spectral range.

The control engine 1010 may be further configured to determine, based on the over time determined illumination distribution, a UV light dose distribution given to the space 1000. For example, the control engine 1010 may determine a UV light dose distribution by, over time, integrating or accumulating values (e.g. pixel values or illumination values) of the illumination distribution. Thus, spatial and/or granular information of the UV light dose given to the space 1000 may be available to the control engine 1010, thereby allowing the control engine 1010 to determine how the UV light dose given to certain parts of a sub-portion 1020 relates to a predetermined UV light dose to be given to each part of the sub-portion 1020 over time. The control engine 1010 may be further configured to determine a UV light dose given to a sub-portion 1020 of the space 1000 using the UV light dose distribution.

The density system may be configured to discard determined positions being older than a predetermined age. Thus, the density system may determine a density distribution based on positions being younger than the predetermined age, thereby allowing the light control system 10 to control the plurality of light sources 1022 such that sub-portions 1020 of the space 1000 which the one or more individuals 1090 have not recently visited are illuminated with light within the ultraviolet spectral range to a relatively lower degree than sub-portions 1020 of the space 1000 which the one or more individuals 1090 have recently visited. Hence, only sub-portions 1020 of the space 1000 that the one or more individuals 1090 have recently visited may be disinfected. The plurality of light sources 1022 may thereby be used more efficiently, whereby an associated wear and tear of each light source 1022 may be reduced. Hence, the plurality of light sources 1022 may continue to function, and thereby be used, for a longer period of time.

The predetermined age may be set based on a UV light dose given to the space 1000. The predetermined age for each sub-portion 1020 of the space 1000 may be set based on a UV light dose given to each corresponding sub-portion 1020 of the space 1000. Thus, the density system may determine a density distribution further based on the UV light dose given to the space 1000, thereby allowing the light control system 10 to control the plurality of light sources 1022 based on the UV light dose given to the space 1000. For example, the control engine 1010 may be allowed to control the plurality of light sources 1022 such that a sub-portion 1020 of the space 1000 which the one or more individuals 1090 have not visited since the sub-portion 1020 was given a UV light dose above a predetermined UV light dose is illuminated with light within the ultraviolet spectral range to a relatively lower degree than sub-portions 1020 of the space 1000 which the one or more individuals 1090 have recently visited. An associated wear and tear of each light source 1022 may thereby be reduced, whereby the plurality of light sources 1022 may continue to function, and thereby be used, for a longer period of time.

It is to be understood that the distance sensor 1038, the object sensor 1037, the pathogen sensor 1039 and the image sensor 1035 may be configured to communicate with the control engine 1010. Such communication may be wired and/or wireless.

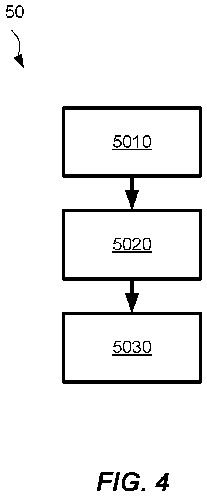

A method 50 for illuminating a space 2000 with light within an ultraviolet spectral range and an example scenario in which the light control system 10 is used will now be described with reference to Fig. 2A, Fig. 2B, and Fig. 4. It is to be understood that the light control system of Fig. 2A may correspond to the light control system 10 of Fig. 1A and Fig. 1B. Hence, the light control system 10 of Fig. 2A may comprise one or more of the technical features shown and described in connection with Fig. 1A and Fig. 1B even though not explicitly shown in the example of Fig. 2A. For example, the space 2000 of Fig. 2A comprises a plurality of light sources similar to the example discussed in connection with Fig. 1A, even though the plurality of light sources is not explicitly shown in Fig. 2A. Technical details are left out of Fig. 2A in order to increase the readability of the figure.

In the example of Fig. 2A, a first individual 2090a, a second individual 2090b, and a third individual 2090c are present in the space 2000. Over time, positions Pa, Pb, Pc of the individuals 2090a, 2090b, 2090c are determined 5010, e.g. by a positioning system as discussed in connection to Fig. 1A and Fig. 1B. In the example shown in Fig. 2, the first individual 2090a is stationary in the first sub-portion 2020a of the space 2000, hence the position Pa is substantially constant. The second individual 2090b moves within the third sub-portion 2020c of the space 2000, and the position Pb-1 changes over time to Pb-2. The third individual 2090c moves from the third sub-portion 2020c to the second sub-portion 2020b of the space 2000, and the position Pc-1 changes over time to Pc-2. As is shown in the example of Fig. 2, none of the individuals 2090a, 2090b, 2090c moves to the fourth sub-portion 2020d of the space 2000.

A density distribution 2100 is illustrated in Fig. 2B. The density distribution 2100 of Fig. 2B corresponds to the space 1000 of Fig. 2A. The density distribution 2100 is determined 5020, e.g. by a density system, based on the over time determined positions of the individuals 2090a, 2090b, 2090c in the space 2000. Density values of the density distribution 2100 varies in between the different sub-portions 2020a, 2020b, 2020c, 2020d of the space 2000. In the example shown in Fig. 2A and Fig. 2B, the density values are lowest in the parts of the density distribution 2100 corresponding to the fourth sub-portion 2020d, since none of the individuals 2090a, 2090b, 2090c have been present in it. The highest density values of the density distribution 2100 corresponds to the third sub-portion 2020c, as the second and the third individuals 2090b, 2090c have been present in it. Similarly, as the third individual 2090c moves to the second sub-portion 2020b, density values corresponding to the second sub-portion 2020b are higher than density values corresponding to the fourth sub-portion 2020d, and lower than density values corresponding to the first and third sub-portions 2020a, 2020c. The space 2000 is illuminated 5030 by the plurality of light sources (not shown in Fig. 2A or Fig. 2B) such that a sub-portion 2020 of the space 2000 having a relatively high density is illuminated with light within the ultraviolet spectral range to a relatively higher degree than a sub-portion 2020 of the space 2000 having a relatively low density. Different manners in which the plurality of light sources 1022 may be adjusted is described in connection to Fig. 1A and Fig. 1B, and they may be applied to the scenario shown in Fig. 2A and Fig. 2B. In order to avoid undue repetition, reference is made to the above discussion in connection to Fig. 1A and Fig. 1B.

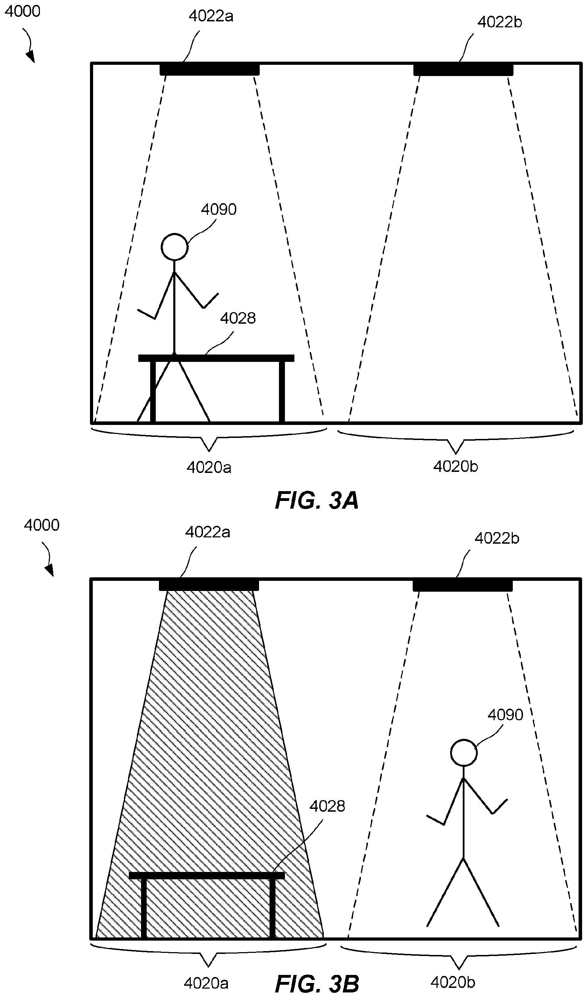

In the specific example shown in Fig. 2A and Fig. 2B, the third sub-portion 2020c is exposed to ultraviolet light to a higher degree than the other sub-portions 2020a, 2020b, 2020d, and the fourth sub-portion 2020d is exposed to ultraviolet light to a lower degree than the other sub-portions 2020a, 2020b, 2020c. The first sub-portion 2020a is exposed to ultraviolet light to a higher degree than the second sub-portion 2020b. As described in connection with Fig. 1A and Fig. 1B, the plurality of light sources 1022 may expose the sub-portions 2020 of the space 2000 to ultraviolet light at a point in time when no individuals 2090 are present in the respective sub-portion 2020, as ultraviolet light may be hazardous for the individuals 2090, which is exemplified in Fig. 3A and Fig. 3B.

In Fig. 3A and Fig. 3B, an individual 4090 is present within a space 4000 comprising a first light source 4022a configured to illuminate a first sub-portion 4020a and a second light source 4022b configured to illuminate a second sub-portion 4020b. In the example of Fig. 3A, the individual 4090 is working by a table 4028 in the first sub-portion 4020a of a space 4000. The density system determines a density distribution, which indicates that density values corresponding to the first sub-portion 4020a are higher than density values corresponding to a second sub-portion 4020b. However, since the individual 4090 is presently present in the first sub-portion 4020a, the control engine (not shown) controls the first light source 4022a such that the first sub-portion 4020a is not exposed to ultraviolet light. At a later point in time, shown in Fig. 3B, the individual 4090 has moved to the second sub-portion 4020b. Since the individual 4090 no longer is present in the first sub-portion 4020a of the space 4000, the control engine controls the first light source 4022a to start exposing the sub-portion 4020a to ultraviolet light. As described previously in connection to Fig. 1A and Fig. 1B, the control engine 1010 may adjust an illumination of the first sub-portion 4020a in a plurality of different manners. To avoid undue repetition, reference is therefore made to the above description.

Now, turning back to Fig. 2A and Fig. 2B; as is described in connection with Fig. 1A and Fig. 1B, different aspects may be accounted for when determining the density distribution 2100. In order to avoid undue repetition, reference is therefore made to the above description. It is further to be understood that the density values of the density distribution 2100 may account for a duration during which the individuals 2090a, 2090b, 2090c have been present at a specific position. For example, in case an individual is stationary at a position (e.g. the first individual 2090a in Fig. 2A), the corresponding density value of the density distribution 2100 may be high. It is further to be understood that the density values of the density distribution 2100 may account for an area of the respective sub-portion. For example, in case the area of a sub-portion is large, the corresponding density values may be lower than for a sub-portion having a smaller area even though individuals have been in the two sub-portions to the same degree.

The person skilled in the art realizes that the present inventive concept by no means is limited to the preferred variants described above. On the contrary, many modifications and variations are possible within the scope of the appended claims.

For example, one or more functions described as being performed by the positioning system and/or the density system may be performed by the control engine 1010. This may, for example, be one or more computations needed to determine the position of the one or more individuals and to determine the density distribution.

As a further example, the control engine may be configured to determine an accumulated amount of light within a visible spectral range the one or more individuals have been exposed to. The control engine may be further configured to control the plurality of light sources such that the one or more individuals are exposed to a predetermined amount of light within the visible spectral range. Such light control system may improve health aspects, e.g. a circadian rhythm of the one or more individuals.

Additionally, variations to the disclosed variants can be understood and effected by the skilled person in practicing the claimed invention, from a study of the drawings, the disclosure, and the appended claims.

## Claims

1. A light control system (10) comprising:
a plurality of light sources (1022, 4022) each configured to emit light within an ultraviolet spectral range, each of the plurality of light sources (1022, 4022) being configured to illuminate a corresponding sub-portion (1020, 2020, 4020) of a space (1000, 2000, 4000),
a positioning system configured to determine positions (P) of one or more individuals (1090, 2090, 4090) within the space (1000, 2000, 4000);
the light control system (10) being **characterized in that** it further comprises
a density system configured to, based on by the positioning system over time determined positions (P) of the one or more individuals (1090, 2090, 4090), determine a density distribution (2100) of the one or more individuals (1090, 2090, 4090) in the space (1000, 2000, 4000); and
a control engine (1010) configured to control the plurality of light sources (1022, 4022) based on the density distribution (2100) such that a sub-portion (1020, 2020, 4020) of the space (1000, 2000, 4000) having a relatively high density is illuminated with light within the ultraviolet spectral range to a relatively higher degree than a sub-portion (1020, 2020, 4020) of the space (1000, 2000, 4000) having a relatively low density.

2. The light control system (10) according to claim 1, wherein the control engine (1010) is further configured to control the plurality of light sources (1022, 4022) based on a present position of the one or more individuals (1090, 2090, 4090) within the space (1000, 2000, 4000), such that a sub-portion (1020, 2020, 4020) of the space (1000, 2000, 4000) in which the one or more individuals (1090, 2090, 4090) are presently present is not exposed to the ultraviolet light emitted by one or more of the plurality of light sources (1022, 4022).

3. The light control system (10) according to claim 1 or 2, wherein a radiant flux of light within the ultraviolet spectral range emitted by each of the plurality of light sources (1022, 4022) is adjustable, and wherein the control engine (1010) is further configured to control the radiant flux of light within the ultraviolet spectral range emitted by the plurality of light sources (1022, 4022) such that each sub-portion (1020, 2020, 4020) of the space (1000, 2000, 4000) is given a respective UV light dose in a predetermined period of time.

4. The light control system (10) according to any preceding claim, further comprising:
a distance sensor (1038) configured to determine a distance (D) between a light source of the plurality of light sources (1022, 4022) and an object (1028) present within the respective sub-portion (1020, 2020, 4020) of the space (1000, 2000, 4000); and
wherein the control engine (1010) is further configured to control the plurality of light sources (1022, 4022) based on the determined distance (D) between each light source and the object (1028) present within each respective sub-portion (1020, 2020, 4020) of the space (1000, 2000, 4000), such that an object at a relatively longer distance from the light source is illuminated by the light source to a relatively higher degree than an object at a relatively shorter distance.

5. The light control system (10) according to any preceding claim, further comprising:
an object sensor (1037) configured to determine a property of an object (1028) present within the space (1000, 2000, 4000), the property being associated with a sensitivity to ultraviolet light and/or an expected contamination level; and
wherein the control engine (1010) is further configured to control the plurality of light sources (1022, 4022) based on the determined property of the object (1028), such that an object having a relatively low sensitivity to ultraviolet light and/or a relatively high expected contamination level is illuminated with light within the ultraviolet spectral range to a relatively higher degree than an object having a relatively high sensitivity to ultraviolet light and/or a relatively low expected contamination level.

6. The light control system (10) according to any preceding claim, further comprising:
a pathogen sensor (1039) configured to dynamically determine a presence of a pathogen within the space (1000, 2000, 4000); and
wherein the control engine (1010) is further configured to control each of the plurality of light sources (1022, 4022) based on the determined presence of the pathogen within each respective sub-portion (1020, 2020, 4020) of the space (1000, 2000, 4000), such that a sub-portion (1020, 2020, 4020) of the space (1000, 2000, 4000) having a determined presence of the pathogen is exposed to light within the ultraviolet spectral range to a relatively higher degree than a sub-portion (1020, 2020, 4020) of the space (1000, 2000, 4000) not having a determined presence of the pathogen.

7. The light control system (10) according to any preceding claim, wherein each light source of the plurality of light sources (1022, 4022) is configured to communicate illumination data to the control engine (1010), the illumination data comprising data of a radiant flux of light within the ultraviolet spectral range emitted by the light source, and wherein the control engine (1010) is further configured to determine, by integrating or accumulating the over time received illumination data, a UV light dose given to each corresponding sub-portion (1020, 2020, 4020) of the space (1000, 2000, 4000).

8. The light control system (10) according to any preceding claim, further comprising:
an image sensor (1035) arranged to acquire one or more images of the space (1000, 2000, 4000); and
wherein the control engine (1010) is further configured to:
using an image acquired by the image sensor (1035), determine, within the space (1000, 2000, 4000), an illumination distribution of light within the ultraviolet spectral range; and
determine, based on the over time determined illumination distribution, a UV light dose distribution given to the space (1000, 2000, 4000).

9. The light control system (10) according to any preceding claim, wherein the density system is configured to discard determined positions (P) being older than a predetermined age.

10. The light control system (10) according to claim 9, wherein the predetermined age is set based on a UV light dose given to the space (1000, 2000, 4000).

11. The light control system (10) according to claim 9 or 10, wherein the predetermined age for each sub-portion (1020, 2020, 4020) of the space (1000, 2000, 4000) is set based on a UV light dose given to each corresponding sub-portion (1020, 2020, 4020) of the space (1000, 2000, 4000).

12. A method (50) for illuminating a space (1000, 2000, 4000) with light within an ultraviolet spectral range using a plurality of light sources (1022, 4022) each configured to illuminate a corresponding sub-portion (1020, 2020, 4020) of the space (1000, 2000, 4000), the method (50) comprising:
determining (5010) positions (P) of one or more individuals (1090, 2090, 4090) within the space (1000, 2000, 4000);
the method being **characterized in** further comprising determining (5020) a density distribution (2100) based on the over time determined positions (P) of the one or more individuals (1090, 2090, 4090) in the space (1000, 2000, 4000); and
illuminating (5030) the space (1000, 2000, 4000) by the plurality of light sources (1022, 4022) based on the density distribution (2100) such that a sub-portion (1020, 2020, 4020) of the space (1000, 2000, 4000) having a relatively high density is illuminated with light within the ultraviolet spectral range to a relatively higher degree than a sub-portion (1020, 2020, 4020) of the space (1000, 2000, 4000) having a relatively low density.

## Patentansprüche

1. Lichtsteuerungssystem umfassend:
eine Vielzahl an Lichtquellen (1022, 4022), von denen jede dazu konfiguriert ist, Licht innerhalb eines ultravioletten Spektralbereichs abzustrahlen, wobei jede der Vielzahl an Lichtquellen (1022, 4022) dazu konfiguriert ist, einen entsprechenden Teilabschnitt (1020, 2020, 4020) eines Raums (1000, 2000, 4000) zu beleuchten;
ein Positioniersystem, das dazu konfiguriert ist, Positionen (P) eines oder mehrerer Individuen (1090, 2090, 4090) innerhalb des Raums (1000, 2000, 4000) zu bestimmen;
wobei das Lichtsteuerungssystem (10) **dadurch gekennzeichnet ist, dass** es ferner Folgendes umfasst:
ein Dichtesystem, das dazu konfiguriert ist, basierend auf von dem Positioniersystem über Zeit bestimmten Positionen (P) des einen oder der mehreren Individuen (1090, 2090, 4090) eine Dichteverteilung (2100) des einen oder der mehreren Individuen (1090, 2090, 4090) in dem Raum (1000, 2000, 4000) zu bestimmen; und
einen Steuermotor (1010), der dazu konfiguriert ist, die Vielzahl an Lichtquellen (1022, 4022) basierend auf der Dichteverteilung (2100) derart zu steuern, dass ein Teilabschnitt (1020, 2020, 4020) des Raums (1000, 2000, 4000), der eine relativ hohe Dichte aufweist, mit Licht innerhalb des ultravioletten Spektralbereichs zu einem relativ höheren Grad beleuchtet wird als ein Teilabschnitt (1020, 2020, 4020) des Raums (1000, 2000, 4000), der eine relativ niedrige Dichte aufweist.

2. Lichtsteuerungssystem (10) nach Anspruch 1, wobei der Steuermotor (1010) ferner dazu konfiguriert ist, die Vielzahl an Lichtquellen (1022, 4022) basierend auf einer vorhandenen Position des einen oder der mehreren Individuen (1090, 2090, 4090) innerhalb des Raums (1000, 2000, 4000) derart zu steuern, dass ein Teilabschnitt (1020, 2020, 4020) des Raums (1000, 2000, 4000), in dem das eine oder die mehreren Individuen (1090, 2090, 4090) aktuell vorhanden sind, nicht dem ultravioletten Licht ausgesetzt wird, das von einer oder mehreren der Vielzahl an Lichtquellen (1022, 4022) abgestrahlt wird.

3. Lichtsteuerungssystem (10) nach Anspruch 1 oder 2, wobei ein Strahlungslichtstrom innerhalb des ultravioletten Spektralbereichs, der von jeder der Vielzahl an Lichtquellen (1022, 4022) abgestrahlt wird, einstellbar ist und wobei der Steuermotor (1010) ferner dazu konfiguriert ist, den Strahlungslichtstrom innerhalb des ultravioletten Spektralbereichs, der von der Vielzahl an Lichtquellen (1022, 4022) abgestrahlt wird, derart zu steuern, dass an jeden Teilabschnitt (1020, 2020, 4020) des Raums (1000, 2000, 4000) eine entsprechende UV-Licht-Dosis in einem vorbestimmten Zeitraum abgegeben wird.

4. Lichtsteuerungssystem (10) nach einem der vorhergehenden Ansprüche, ferner umfassend:
einen Distanzsensor (1038), der dazu konfiguriert ist, eine Distanz (D) zwischen einer Lichtquelle der Vielzahl an Lichtquellen (1022, 4022) und einem Objekt (1028) zu bestimmen, dass innerhalb des entsprechenden Teilabschnitts (1020, 2020, 4020) des Raums (1000, 2000, 4000) vorhanden ist; und
wobei der Steuermotor (1010) ferner dazu konfiguriert ist, die Vielzahl an Lichtquellen (1022, 4022) basierend auf der bestimmten Distanz (D) zwischen jeder Lichtquelle und dem Objekt (1028), das innerhalb jedes entsprechenden Teilabschnitts (1020, 2020, 4020) des Raums (1000, 2000, 4000) vorhanden ist, derart zu steuern, dass ein Objekt mit einer relativ längeren Distanz zu der Lichtquelle von der Lichtquelle zu einem relativ höheren Grad beleuchtet wird als ein Objekt mit einer relativ kürzeren Distanz.

5. Lichtsteuerungssystem (10) nach einem der vorhergehenden Ansprüche, ferner umfassend:
einen Objektsensor (1037), der dazu konfiguriert ist, eine Eigenschaft eines Objekts (1028) zu bestimmen, das innerhalb des Raums (1000, 2000, 4000) vorhanden ist, wobei die Eigenschaft mit einer Empfindlichkeit gegenüber ultraviolettem Licht und/oder einem erwarteten Verschmutzungsgrad verbunden ist; und
wobei der Steuermotor (1010) ferner dazu konfiguriert ist, die Vielzahl an Lichtquellen (1022, 4022) basierend auf der bestimmten Eigenschaft des Objekts (1028) derart zu steuern, dass ein Objekt, das eine relativ niedrige Empfindlichkeit gegenüber ultraviolettem Licht und/oder einen relativ hohen erwarteten Verschmutzungsgrad aufweist, mit Licht innerhalb des ultravioletten Spektralbereichs zu einem relativ höheren Grad beleuchtet wird als ein Objekt, das eine relativ hohe Empfindlichkeit gegenüber ultraviolettem Licht und/oder einen relativ niedrigen erwarteten Verschmutzungsgrad aufweist.

6. Lichtsteuerungssystem (10) nach einem der vorhergehenden Ansprüche, ferner umfassend:
einen Pathogensensor (1039), der dazu konfiguriert ist, ein Vorhandensein eines Pathogens innerhalb des Raums (1000, 2000, 4000) dynamisch zu bestimmen; und
wobei der Steuermotor (1010) ferner dazu konfiguriert ist, jede der Vielzahl an Lichtquellen (1022, 4022) basierend auf dem bestimmten Vorhandensein des Pathogens innerhalb jedes entsprechenden Teilabschnitts (1020, 2020, 4020) des Raums (1000, 2000, 4000) derart zu steuern, dass ein Teilabschnitt (1020, 2020, 4020) des Raums (1000, 2000, 4000), der ein bestimmtes Vorhandensein des Pathogens aufweist, Licht innerhalb des ultravioletten Spektralbereichs zu einem relativ höheren Grad ausgesetzt wird als ein Teilabschnitt (1020, 2020, 4020) des Raums (1000, 2000, 4000), der kein bestimmtes Vorhandensein des Pathogens aufweist.

7. Lichtsteuerungssystem (10) nach einem der vorhergehenden Ansprüche, wobei jede Lichtquelle der Vielzahl an Lichtquellen (1022, 4022) dazu konfiguriert ist, Beleuchtungsdaten an den Steuermotor (1010) zu kommunizieren, wobei die Beleuchtungsdaten Daten eines Strahlungslichtstroms innerhalb des ultravioletten Spektralbereichs umfassen, der von der Lichtquelle abgestrahlt wird, und wobei der Steuermotor (1010) ferner dazu konfiguriert ist, durch Integrieren oder Akkumulieren der über Zeit erhaltenen Beleuchtungsdaten eine UV-Licht-Dosis zu bestimmen, die an jeden entsprechenden Teilabschnitt (1020, 2020, 4020) des Raums (1000, 2000, 4000) abgegeben wird.

8. Lichtsteuerungssystem (10) nach einem der vorhergehenden Ansprüche, ferner umfassend:
einen Bildsensor (1035), der dazu angeordnet ist, ein oder mehrere Bilder des Raums (1000, 2000, 4000) zu erfassen; und
wobei der Steuermotor (1010) ferner zu Folgendem konfiguriert ist:
Verwenden eines Bildes, das von dem Bildsensor (1035) erfasst wurde, Bestimmen, innerhalb des Raums (1000, 2000, 4000), einer Beleuchtungsverteilung von Licht innerhalb des ultravioletten Spektralbereichs; und
Bestimmen, basierend auf der über Zeit bestimmten Beleuchtungsverteilung, einer UV-Licht-Dosis-Verteilung, die an den Raum (1000, 2000, 4000) abgegeben wird.

9. Lichtsteuerungssystem (10) nach einem der vorhergehenden Ansprüche, wobei das Dichtesystem dazu konfiguriert ist, bestimmte Positionen (P), die älter sind als ein vorbestimmtes Alter, zu verwerfen.

10. Lichtsteuerungssystem (10) nach Anspruch 9, wobei das vorbestimmte Alter basierend auf einer UV-Licht-Dosis eingestellt wird, die an den Raum (1000, 2000, 4000) abgegeben wird.

11. Lichtsteuerungssystem (10) nach Anspruch 9 oder 10, wobei das vorbestimmte Alter für jeden Teilabschnitt (1020, 2020, 4020) des Raums (1000, 2000, 4000) basierend auf einer UV-Licht-Dosis eingestellt wird, die an jeden entsprechenden Teilabschnitt (1020, 2020, 4020) des Raums (1000, 2000, 4000) abgegeben wird.

12. Verfahren (50) zum Beleuchten eines Raums (1000, 2000, 4000) mit Licht innerhalb eines ultravioletten Spektralbereichs unter Verwendung einer Vielzahl an Lichtquellen (1022, 4022), von denen jede dazu konfiguriert ist, einen entsprechenden Teilabschnitt (1020, 2020, 4020) des Raums (1000, 2000, 4000) zu beleuchten, wobei das Verfahren (50) Folgendes umfasst:
Bestimmen (5010) von Positionen (P) eines oder mehreren Individuen (1090, 2090, 4090) innerhalb des Raums (1000, 2000, 4000);
wobei das Verfahren **dadurch gekennzeichnet** ist, das es ferner Folgendes umfasst:
Bestimmen (5020) einer Dichteverteilung (2100) basierend auf den über Zeit bestimmten Positionen (P) des einen oder der mehreren Individuen (1090, 2090, 4090) in dem Raum (1000, 2000, 4000); und
Beleuchten (5030) des Raums (1000, 2000, 4000) mit der Vielzahl an Lichtquellen (1022, 4022) basierend auf der Dichteverteilung (2100) derart, dass ein Teilabschnitt (1020, 2020, 4020) des Raums (1000, 2000, 4000), der eine relativ hohe Dichte aufweist, mit Licht innerhalb des ultravioletten Spektralbereichs zu einem relativ höheren Grad beleuchtet wird als ein Teilabschnitt (1020, 2020, 4020) des Raums (1000, 2000, 4000), der eine relativ niedrige Dichte aufweist.

## Revendications

1. Système de commande de lumière (10) comprenant :
une pluralité de sources de lumière (1022, 4022) configurées chacune pour émettre de la lumière dans une plage spectrale ultraviolette, chacune de la pluralité de sources de lumière (1022, 4022) étant configurée pour éclairer une sous-partie correspondante (1020, 2020, 4020) d'un espace (1000, 2000, 4000) ;
un système de positionnement configuré pour déterminer la position (P) d'un ou plusieurs individus (1090, 2090, 4090) dans l'espace (1000, 2000, 4000) ;
le système de commande de lumière (10) étant **caractérisé en ce qu'**il comprend en outre
un système de densité configuré pour, sur la base des positions (P) déterminées au fil du temps par le système de positionnement du ou des individus (1090, 2090, 4090), déterminer une distribution de densité (2100) des un ou plusieurs individus (1090, 2090, 4090) dans l'espace (1000, 2000, 4000) ; et
un moteur de commande (1010) configuré pour commander la pluralité de sources de lumière (1022, 4022) sur la base de la distribution de densité (2100) de telle sorte qu'une sous-partie (1020, 2020, 4020) de l'espace (1000, 2000, 4000) ayant une densité relativement élevée est éclairée avec une lumière dans la plage spectrale ultraviolette à un degré relativement plus élevé qu'une sous-partie (1020, 2020, 4020) de l'espace (1000, 2000, 4000) ayant une densité relativement faible.

2. Système de commande de lumière (10) selon la revendication 1, dans lequel le moteur de commande (1010) est en outre configuré pour commander la pluralité de sources de lumière (1022, 4022) en fonction d'une position actuelle d'un ou plusieurs individus (1090, 2090, 4090) dans l'espace (1000, 2000, 4000), de sorte qu'une sous-partie (1020, 2020, 4020) de l'espace (1000, 2000, 4000) dans laquelle un ou plusieurs individus (1090, 2090, 4090) sont actuellement présents ne soit pas exposée à la lumière ultraviolette émise par une ou plusieurs de la pluralité de sources de lumière (1022, 4022).

3. Système de commande de lumière (10) selon la revendication 1 ou 2, dans lequel un flux de rayonnement de lumière dans la plage spectrale ultraviolette émis par chacune de la pluralité de sources de lumière (1022, 4022) est réglable, et dans lequel le moteur de commande (1010) est en outre configuré pour commander le flux de rayonnement de lumière dans la plage spectrale ultraviolette émis par la pluralité de sources lumière (1022, 4022) de telle sorte que chaque sous-partie (1020, 2020, 4020) de l'espace (1000, 2000, 4000) reçoive une dose de lumière UV respective dans une période de temps prédéterminée.

4. Système de commande de lumière (10) selon l'une quelconque des revendications précédentes, comprenant en outre :
un capteur de distance (1038) configuré pour déterminer une distance (D) entre une source de lumière de la pluralité de sources de lumière (1022, 4022) et un objet (1028) présent dans la sous-partie respective (1020, 2020, 4020) de l'espace (1000, 2000, 4000) ; et
dans lequel le moteur de commande (1010) est en outre configuré pour commander la pluralité de sources de lumière (1022, 4022) sur la base de la distance déterminée (D) entre chaque source de lumière et l'objet (1028) présent dans chaque sous-partie respective (1020, 2020, 4020) de l'espace (1000, 2000, 4000), de telle sorte qu'un objet à une distance relativement plus longue de la source lumière soit éclairé par la source lumière à un degré relativement plus élevé qu'un objet à une distance relativement plus courte.

5. Système de commande de lumière (10) selon l'une quelconque des revendications précédentes, comprenant en outre :
un capteur d'objet (1037) configuré pour déterminer une propriété d'un objet (1028) présent dans l'espace (1000, 2000, 4000), la propriété étant associée à une sensibilité à la lumière ultraviolette et/ou à un niveau de contamination attendu ; et
dans lequel le moteur de commande (1010) est en outre configuré pour commander la pluralité de sources de lumière (1022, 4022) sur la base de la propriété déterminée de l'objet (1028), de sorte qu'un objet ayant une sensibilité relativement faible à la lumière ultraviolette et/ou un niveau de contamination attendu relativement élevé soit éclairé par une lumière dans la plage spectrale ultraviolette à un degré relativement plus élevé qu'un objet ayant une sensibilité relativement élevée à la lumière ultraviolette et/ou un niveau de contamination attendu relativement faible.

6. Système de commande de lumière (10) selon l'une quelconque des revendications précédentes, comprenant en outre :
un capteur de pathogène (1039) configuré pour déterminer dynamiquement la présence d'un pathogène dans l'espace (1000, 2000, 4000) ; et
dans lequel le moteur de commande (1010) est en outre configuré pour commander chacune de la pluralité de sources de lumière (1022, 4022) sur la base de la présence déterminée du pathogène dans chaque sous-partie respective (1020, 2020, 4020) de l'espace (1000, 2000, 4000), de telle sorte qu'une sous-partie (1020, 2020, 4020) de l'espace (1000, 2000, 4000) ayant une présence déterminée du pathogène est exposée à la lumière dans la plage spectrale ultraviolette à un degré relativement plus élevé qu'une sous-partie (1020, 2020, 4020) de l'espace (1000, 2000, 4000) n'ayant pas de présence déterminée du pathogène.

7. Système de commande de lumière (10) selon l'une quelconque des revendications précédentes, dans lequel chaque source de lumière de la pluralité de sources de lumière (1022, 4022) est configurée pour communiquer des données d'éclairage au moteur de commande (1010), les données d'éclairage comprenant des données d'un flux de rayonnement de lumière dans la plage spectrale ultraviolette émise par la source de lumière, et dans lequel le moteur de commande (1010) est en outre configuré pour déterminer, en intégrant ou en accumulant les données d'éclairage reçues au fil du temps, une dose de lumière UV donnée à chaque sous-partie (1020, 2020, 4020) correspondante de l'espace (1000, 2000, 4000).

8. Système de commande de lumière (10) selon l'une quelconque des revendications précédentes, comprenant en outre :
un capteur d'image (1035) agencé pour acquérir une ou plusieurs images de l'espace (1000, 2000, 4000) ; et
dans lequel le moteur de commande (1010) est en outre configuré pour :
en utilisant une image acquise par le capteur d'image (1035), déterminer, dans l'espace (1000, 2000, 4000), une distribution d'éclairage de la lumière dans la plage spectrale ultraviolette ; et
déterminer, sur la base de la distribution d'éclairage déterminée au fil du temps, une distribution de dose de lumière UV donnée à l'espace (1000, 2000, 4000).

9. Système de commande de lumière (10) selon l'une quelconque des revendications précédentes, dans lequel le système de densité est configuré pour éliminer les positions déterminées (P) qui sont plus anciennes qu'un âge prédéterminé.

10. Système de commande de lumière (10) selon la revendication 9, dans lequel l'âge prédéterminé est défini sur la base d'une dose de lumière UV donnée à l'espace (1000, 2000, 4000).

11. Système de commande de lumière (10) selon la revendication 9 ou 10, dans lequel l'âge prédéterminé pour chaque sous-partie (1020, 2020, 4020) de l'espace (1000, 2000, 4000) est défini sur la base d'une dose de lumière UV donnée à chaque sous-partie (1020, 2020, 4020) correspondante de l'espace (1000, 2000, 4000).

12. Procédé (50) d'éclairage d'un espace (1000, 2000, 4000) avec une lumière dans une plage spectrale ultraviolette à l'aide d'une pluralité de sources de lumière (1022, 4022), chacune étant configurée pour éclairer une sous-partie (1020, 2020, 4020) correspondante de l'espace (1000, 2000, 4000), le procédé (50) comprenant :
la détermination (5010) des positions (P) d'un ou plusieurs individus (1090, 2090, 4090) dans l'espace (1000, 2000, 4000) ;
le procédé étant **caractérisé en ce qu'**il comprend en outre la détermination (5020) d'une distribution de densité (2100) sur la base des positions (P) déterminées au fil du temps des un ou plusieurs individus (1090, 2090, 4090) dans l'espace (1000, 2000, 4000) ; et
l'éclairage (5030) de l'espace (1000, 2000, 4000) par la pluralité de sources de lumière (1022, 4022) sur la base de la distribution de densité (2100) de telle sorte qu'une sous-partie (1020, 2020, 4020) de l'espace (1000, 2000, 4000) ayant une densité relativement élevée est éclairée avec une lumière dans la plage spectrale ultraviolette à un degré relativement plus élevé qu'une sous-partie (1020, 2020, 4020) de l'espace (1000, 2000, 4000) ayant une densité relativement faible.
